# EUROPEAN PATENT APPLICATION

(11) **EP 4 556 464 A1**
(43) Date of publication of application: **21.05.2025**
(21) Application number: 23838892.0
(22) Date of filing: 10.07.2023
(51) Int. Cl.: C07C 403/20, A61K 31/203, A61P 17/00, A61P 35/00

(54) **TRETINOIN ALCOHOL-AMINE COMPOUND, PREPARATION METHOD THEREFOR AND USE THEREOF**

(30) Priority: 11.07.2022 CN 202210853741
(71) Applicant: NANJING INDETEK LABORATORY CO., LTD., Nanjing, Jiangsu 211106 (CN); Fudan University, Shanghai 201203 (CN)
(72) Inventor: LIU, Zhenyun, Nanjing, Jiangsu 211106 (CN); HU, Tao, Nanjing, Jiangsu 211106 (CN); LU, Xiqiang, Nanjing, Jiangsu 211106 (CN); QI, Jianping, Shanghai 201203 (CN); WU, Wei, Shanghai 201203 (CN); WU, Yubo, Nanjing, Jiangsu 211106 (CN); CHEN, Lei, Nanjing, Jiangsu 211106 (CN)
(74) Representative: Gille Hrabal Partnerschaftsgesellschaft mbB Patentanwälte
(86) International application number: PCT/CN2023/106535
(87) International publication number: WO 2024/012400

(57) **Abstract**

The present invention relates to a retinoic acid-alcohol amine complex consisting of retinoic acid and an alcohol amine compound. The complex is in liquid form at room temperature, has good water solubility/water dilution resistance and good skin penetration effect, and overcomes the limitation of retinoic acid compounds in the application field of drug products in the prior art, showing a wide prospect in medicine.

## Description

This application claims priority to prior Application No. 202210853741.9, filed to China National Intellectual Property Administration on July 11, 2022 and entitled "RETINOIC ACID-ALCOHOL AMINE COMPLEX, PREPARATION METHOD AND APPLICATION THEREOF", which is incorporated herein by reference in its entirety.

### TECHNICAL FIELD

The present invention relates to pharmaceutical chemistry, and in particular relates to a retinoic acid-alcohol amine complex, a preparation method and application thereof.

### BACKGROUND OF THE INVENTION

Vitamin A and its metabolites play an important role in the human body and have been widely used in the treatment of various diseases. For example, retinoic acid, with the molecular formula of C₂₀H₂₈O₂, is a metabolic intermediate product of vitamin A in vivo. It mainly affects bone growth and promotes metabolic effects such as epithelial cell proliferation, differentiation, and keratolysis. The retinoic acid is mainly used for treating diseases such as common acnes, hyperpigmentation, skin photoaging, psoriasis, ichthyosis, lichen planus, pityriasis rubra pilaris, keratosis follicularis, squamous cell carcinoma, and melanoma. Oral retinoic acid can also be used to treat acute promyelocytic leukemia.

The retinoic acid is fat-soluble and almost insoluble in water, and thus has poor solubility in a drug product and low dissolution in the body, making its drug product limited in medicinal use. In addition, due to the barrier function of stratum corneum of skin, a conventional external drug product of retinoic acid, when in use, has only a small amount of ingredients that can penetrate through the barrier to exert their effects, making it difficult to keep an effective therapeutic concentration; however, if the dosage of retinoic acid is increased, greater skin irritation will be caused, such as redness, stinging, edema and other inflammatory symptoms to different extents. In view of the promising application of retinoic acid in treatment of skin diseases, it is necessary to develop it into a medicinal form suitable for skin diseases.

### SUMMARY OF THE INVENTION

In order to ameliorate the technical problem existing in the prior art, in a first aspect, the present invention provides a retinoic acid-alcohol amine complex, the complex comprises a retinoic acid and an alcohol amine compound.

According to an embodiment of the present invention, the alcohol amine compound is selected from formula I as follows: 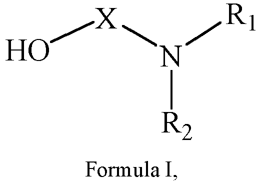 wherein X is selected from C1-6 alkyl, preferably, from -CH₂-CH₂-, -CH₂-CH₂-CH₂- and 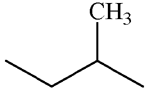 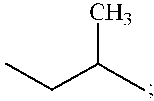 and R₁ and R₂ are each independently selected from H, C₁₋₆ alkyl and C₁₋₆ alkyl-OH.

According to an embodiment of the present invention, the formula I is selected from diethanolamine, triethanolamine, monoethanolamine(2-hydroxyethylamine), N-methyldiethanolamine, n-propanolamine, isopropanolamine, diisopropanolamine, triisopropanolamine, n-butanolamine, dibutolamine, and isobutanolamine.

According to an embodiment of the present invention, the molar ratio of the alcohol amine compound to the retinoic acid is (1-100):1, for example, 2:1, 2.5:1, 3:1, 3.5:1, 4:1, 4.5:1, 5:1, 5.5:1, 6:1,, 6.5:1, 7:1, or (1-10):1, more preferably, (1-5):1.

According to an embodiment of the present invention, the complex is in liquid form at room temperature. In the complex, the retinoic acid can exist partly or wholly in anionic form, and the alcohol amine compound can exist partly or wholly in cationic form. In some embodiments, in the complex, the retinoic acid exists partly in molecular form, and/or the alcohol amine compound exists partly in molecular form.

In some embodiments, in the complex, the retinoic acid exists in [A⁻] form as follows:

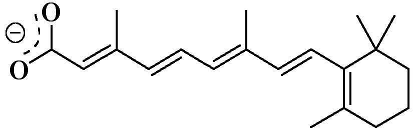

In some embodiments, in the complex, the alcohol amine compound exists in [B⁺] form as shown in formula II as follows: 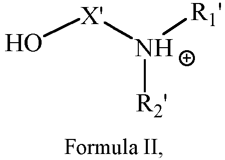 wherein X' is selected from C1-6 alkyl, preferably, from -CH₂-CH₂-, -CH₂-CH₂-CH₂- and 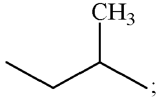 and R_{1'} and R₂' are each independently selected from H, C₁₋₆ alkyl and C₁₋₆ alkyl-OH.

According to an embodiment of the present invention, the formula II is selected from diethanolamine cation, triethanolamine cation, monoethanolamine cation (i.e., 2-hydroxyethylamine cation), N-methyldiethanolamine cation, n-propanolamine cation, isopropanolamine cation, diisopropanolamine cation, triisopropanolamine cation, n-butanolamine cation, dibutolamine cation, and isobutanolamine cation.

In some embodiments, the compound is [A⁻] [B⁺]_{X}, wherein x can be selected from 1-10, for example, 1, 1.5, 2, 2.5, 3, 3.5, 4, 4.5, 5, 5.5, 6, 6.5, and 7.

In a second aspect, the present invention provides a preparation method for the complex. The preparation method includes the step of: reacting the retinoic acid with the alcohol amine compound to obtain the complex.

According to an embodiment of the present invention, the molar ratio of the alcohol amine compound to the retinoic acid is (1-100):1, for example, 2:1, 2.5:1, 3:1, 3.5:1, 4:1, 4.5:1, 5:1, 5.5:1, 6:1, 6.5:1, 7:1, or (1-10): 1, and preferably (1-5): 1.

According to an embodiment of the present invention, the reacting step may be carried out in a reaction reagent that may be water, or an organic solvent, or a mixed solvent of the organic solvent and the water, and the organic solvent is selected from one or more of ethanol, methanol and acetone. Preferably, the organic solvent is ethanol.

According to an embodiment of the present invention, the volume ratio of organic solvent to water in the mixed solvent is 5:95~95:5. Preferably, in the mixed solvent, the volume ratio of organic solvent to water is 40:60~95:5.

According to an embodiment of the present invention, the reacting step may be carried out by dry grinding.

According to an embodiment of the present invention, the total dosage ratio of the retinoic acid to the reaction reagent is 1 g:5-150 mL, and preferably 1 g:5-20 mL.

According to an embodiment of the present invention, the preparation method includes the steps of:
(1) adding the reaction reagent to the retinoic acid, and stirring to mix;
(2) adding the reaction reagent to the alcohol amine compound, and stirring to mix; and
(3) adding the diluent obtained from step (2) to the mixture obtained from step (1).

According to an embodiment of the present invention, in step (1), the dosage ratio of the retinoic acid to the reaction reagent is 1 g:5-100 mL, and preferably 1 g:5-20 mL; and in the step (2), the dosage ratio of the alcohol amine compound to the reaction reagent is 1 g:0.5-50 mL, preferably 1 g:0.5-5 mL, and more preferably 1 g:0.5-1 mL.

According to an embodiment of the present invention, the preparation method further includes the steps of: after the reaction is completed, removing the reaction reagent and drying in vacuum.

In a third aspect, the present invention further provides a composition including the retinoic acid-alcohol amine complex.

In a fourth aspect, the present invention provides an application of the retinoic acid-alcohol amine complex or the composition in preparation of a drug product, including but not limited to pharmaceutical drug products, cosmetics, care products, and beauty products.

The pharmaceutical drug product is applied to the treatment of skin diseases and non-skin tumors. Preferably, the pharmaceutical drug product is used to treat skin diseases, such as common acnes, hyperpigmentation, skin photoaging, psoriasis, ichthyosis, lichen planus, pityriasis rubra pilaris, keratosis follicularis, squamous cell carcinoma, and melanoma. In some embodiments, the pharmaceutical drug product is applied to the treatment of non-skin tumors, such as acute promyelocytic leukemia, and may also be used for treating gastric cancer, lung cancer, ovarian cancer, cervical cancer, neuroblastic carcinoma, glioma or the like.

According to an embodiment of the present invention, the drug product can be applied topically through the skin, and is selected from, for example, cream, patch, ointment, paste, gel, spray and the like, and the drug product may also be administered orally, i.e., in form of oral drug product, which is selected from, for example, tablet, granule, capsules, oral liquid drug product, pill, suspension, drop pill and the like. The drug product further includes a physiologically acceptable carrier (for example, a biocompatible material), which optionally includes surfactant, excipient, humectant, emulsification promoter, suspending aid, salt or buffer for regulating osmotic pressure, colorant, flavor, stabilizer, sterilizing agent, preservative or other conventional supplements.

According to an embodiment of the present invention, the administration route of the drug product includes, but is not limited to, gastrointestinal administration or non-gastrointestinal administration, wherein the gastrointestinal administration may be oral administration, and the non-gastrointestinal administration may be transdermal administration, or the like.

### Advantageous Effects of the Invention

The present invention has unexpectedly found that retinoic acid-alcohol amine compound may form a complex in liquid form at room temperature, which has good water solubility/water dilution resistance and good skin penetration effect, and overcomes the limitation of retinoic acid compounds in the application field of drug products in the prior art, showing a wide prospect in medicine. The preparation method used in this study is simple and feasible without harsh reaction conditions, and is easy for quality control and production scale-up; and in addition, the solvent used is recoverable for reutilization, which is in line with the concept of green chemistry.

### BRIEF DESCRIPTION OF THE DRAWINGS

Fig. 1 shows an HNMR spectrum of retinoic acid;
Fig. 2 shows an HNMR spectrum of diethanolamine;
Fig. 3 shows an HNMR spectrum of Complex 2[DEA][RA];
Fig. 4 shows IR spectra of retinoic acid, diethanolamine, and Complex 2[DEA][RA];
Fig. 5 shows an IR spectrum of retinoic acid-diethanolamine complex at different molar ratios ([RA]:[DEA] = 1:2, 1:2.5, 1:3 or 1:4);
Fig. 6 shows Raman spectra of retinoic acid, diethanolamine, and Complex 2[DEA][RA];
Fig. 7 shows DVS of Complex 2[DEA][RA];
Fig. 8 shows PLM photos of Complex 2[DEA][RA] before and after DVS;
Fig. 9 shows DSC of Complex 2[DEA][RA];
Fig. 10 shows mDSC of Complex 2[DEA][RA];
Fig. 11 shows characteristics of Complex 2[DEA][RA] with different water contents;
Fig. 12 shows a process of a liquid nitrogen freezing test for Complex 2[DEA][RA];
Fig. 13 shows an HNMR spectrum of Complex 2.5[DEA][RA];
Fig. 14 shows an HNMR spectrum of Complex 3[DEA][RA]; and
Fig. 15(a) indicates that a complex cannot be formed from 7 [TEA][RA] (solid precipitation, turbid state); and
Fig. 15(b) indicates a complex can be formed from 8 [TEA][RA] (transparent reddish-brown liquid).

### DETAILED DESCRIPTION OF THE INVENTION

The technical solutions of the present invention will be further described in detail below in conjunction with specific embodiments. It should be understood that the following embodiments are only for a purpose of exemplary illustration and explanation of the present invention, and should not be construed as limiting the protection scope of the present invention. All techniques achieved on the basis of the above-mentioned solutions of the present invention shall be covered by the scope of the present invention.

Unless otherwise stated, the raw materials and reagents used in the following embodiments are commercially available, or may be prepared by known methods.

### Instrument & Testing Conditions:

| Test Item | Device | Test condition |
|---|---|---|
| HNMR spectrum | Bruker 400M NMR Spectrometer | DMSO-d6 as solvent |
| HNMR spectrum | Bruker 600M NMR Spectrometer | DMSO-d6 as solvent |
| Infrared spectrum | Brucker Vertex 70 | Attenuated total reflection (ATR) mode; collected at room temperature; KBr beam splitter; wavenumber range of the test: 4000~600 cm⁻¹; and resolution: 4 cm⁻¹ |
| Infrared spectrum | ThermoFisher Nicolet FT-IR | Attenuated total reflection (ATR) mode; collected at room temperature; KBr beam splitter; wavenumber range of the test: 4000~600 cm⁻¹; and resolution: 4 cm⁻¹ |
| Raman spectrum | HORIBA HR800 | laser wavelength: 633 nm; grating wavelength: 600 nm |
| Dynamic vapor sorption test | SMS Intrinsic dynamic moisture adsorber | Temperature: 25°C; air flow: nitrogen; minimum equilibrium time: 10 min; maximum equilibrium time: 180 min; resolution: 0.002%/min; and humidity range: 0%RH-95%RH-0%RH |
| Polarized microscopy | Carl Zeiss Axio Scope A1 | Shoot at room temperature |

Unless otherwise stated, the percentage content "%" in the following embodiment represents the mass percentage content.

### Example 1. Preparation of Retinoic Acid-Diethanolamine Complex

### 1.1 Raw materials and reagents: retinoic acid (RA) (AR, Shanghai Aladdin Biochemical Technology Co., Ltd.), diethanolamine (DEA) (AR, Sinopharm Chemical Reagent Co., Ltd.), ethanol (AR, Sinopharm Chemical Reagent Co., Ltd.).

### 1.2 Preparation process of retinoic acid-diethanolamine complex

### 1.2.1 Preparation Method 1

### Prescription

| Material | Amount (DEA/RA molar ratio: 2:1) | Solvent | Amount of solvent |
|---|---|---|---|
| Retinoic acid (RA) | 6.0g | Ethanol | 30ml~60ml |
| Diethanolamine (DEA) | 4.2g | Ethanol | 5ml |

(1) Retinoic acid was weighed according to the prescribed amount and placed in a round-bottom flask, and ethanol was added and then stirred for mixing; the retinoic acid was not completely dissolved and was in a suspended turbid state; and the round-bottom flask was wrapped in an aluminum foil for shading.
(2) Diethanolamine was weighed according to the prescribed amount and placed in a container wrapped in an aluminum foil, and ethanol was added and then stirred for dilution.

The diethanolamine dilution obtained in step (2) was added dropwise, over stirring, to the mixture obtained in step (1); and the container was washed with about 5 ml of ethanol, which was then added to the flask and stirred.

After dropwise adding was completed, the solution should be in a clear orange-red state, and stirring is continued for 4 hours.

The solvent was removed using a rotary evaporator: vacuum degree (gauged): -0.095 Mpa; programmed heating method was used to prevent ethanol boiling and splashing; rotary evaporation was carried out for 1 h at 45°C, continued for another 1 h at 50°C, and then, continued for 2 h at 60°C. The resulting viscous reddish-brown liquid was extracted while it was hot, and then was continued to be dried in vacuum for 48 h in a vacuum drying oven at 60°C. The resulting sample was sealed and stored in a dark place.

### 1.2.2 Preparation Method 2

### Prescription:

| Material | Amount (DEA/RA molar ratio: 2:1) | Solvent | Amount of solvent |
|---|---|---|---|
| Retinoic acid (RA) | 6.0g | Ethanol:water = 50:50 | 50ml |
| Diethanolamine (DEA) | 4.2g | Ethanol:water = 50:50 | 5ml |

### Process:

Retinoic acid was weighed according to the prescribed amount and placed in a round-bottom flask, and ethanol/water was added and then stirred for mixing; the retinoic acid was not completely dissolved and was in a suspended turbid state; and the round-bottom flask was wrapped in an aluminum foil for shading.

Diethanolamine was weighed according to the prescribed amount and placed in a container wrapped in an aluminum foil, and ethanol/water was added and then stirred for dilution.

The diethanolamine dilution was added dropwise, over stirring, to the round-bottom flask containing the retinoic acid mixture; and the container was washed with about 5 ml of ethanol/water, which was then added to the flask and stirred. After dropwise adding was completed, the solution should be in a clear orange-red state, and stirring is continued for 4 hours. The solvent was removed using a rotary evaporator. The resulting viscous reddish-brown liquid was extracted while it was hot, and then was continued to be dried in vacuum for 48 h in a vacuum drying oven at 60°C. The resulting sample was sealed and stored in a dark place.

### 1.2.3 Preparation Method 3

### Prescription:

| Material | Amount | Solvent | Amount of solvent |
|---|---|---|---|
| Retinoic acid (RA) | 6.0g | \ | \ |
| Diethanolamine (DEA) | 4.2g | Water | 50ml |

### Process:

Diethanolamine was weighed according to the prescribed amount and placed in a round-bottom flask, and the round-bottom flask was wrapped in an aluminum foil for shading.

A prescribed amount of water was added to the round-bottom flask, and stirred till uniformity. A prescribed amount of powdery retinoic acid was weighed, and then carefully add portionwise to the flask over stirring. After the resulting mixture became clear by stirring, stirring was continued for another 4 h. The solvent was removed using a rotary evaporator. The resulting viscous reddish-brown liquid was extracted while it was hot, and then was continued to be dried in vacuum for 48 h in a vacuum drying oven at 60°C. The resulting sample was sealed and stored in a dark place.

### 1.2.4 Preparation Method 4

### Prescription:

| Material | Amount (DEA/RA molar ratio: 3:1) |
|---|---|
| Retinoic acid (RA) | 6.0g |
| Diethanolamine (DEA) | 6.3g |

### Process:

The test procedure should be carried out in the dark. Retinoic acid was weighted according to the prescribed amount and placed in a crucible; and then, a prescribed amount of diethanolamine was added. The two materials were carefully mixed well using a fine pestle. The crucible was heated in an electric heating jacket to maintain the temperature at 60~80°C. Stirring and grinding were continued till the formation of a viscous reddish-brown liquid. The liquid was transferred to a light-resistant container while it was hot, cooled and sealed for storage.

### 1.3 Preparation of retinoic acid-diethanolamine complexes at different ratios

Referring to the preparation process of 1.2.1, the retinoic acid and the diethanolamine at different ratios (as shown in the table below) were used to prepare the complex, and the results showed that the complex (clear reddish-brown liquid at room temperature) was achievable as long as the molar ratio of the retinoic acid to the diethanolamine was 2:1~10:1.

**Table A-1: Complexes formed from retinoic acid and diethanolamine at different ratios**

| **Name** | **Molar ratio [DEA]: [RA]** | **State at room temperature** |
|---|---|---|
| 2[DEA][RA] | 2:1 | |
| 2.5[DEA][RA] | 2.5:1 | |
| 3[DEA][RA] | 3:1 | |
| 4[DEA][RA] | 4:1 | |
| 5[DEA][RA] | 5:1 | Transparent reddish-brown liquid |
| 6[DEA][RA] | 6:1 | |
| 7[DEA][RA] | 7:1 | |
| 8[DEA][RA] | 8:1 | |
| 9[DEA][RA] | 9:1 | |
| 10[DEA][RA] | 10:1 | |

### 1.4 Characterization of retinoic acid-diethanolamine complex

Test objective: Through the detection and analysis of the nuclear magnetic resonance hydrogen (HNMR) spectra, infrared spectra and Raman spectra of the raw materials and the retinoic acid-diethanolamine complex, it can be determined that the product formed from the retinoic acid and the diethanolamine was not a simple physical mixture, but with intermolecular interaction and with ionic bonds and hydrogen bonds, and this constitutes the chemical basis of the special physiochemical properties of the retinoic acid complex.

### 1.4.1 Nuclear magnetic resonance hydrogen (HNMR) spectrum

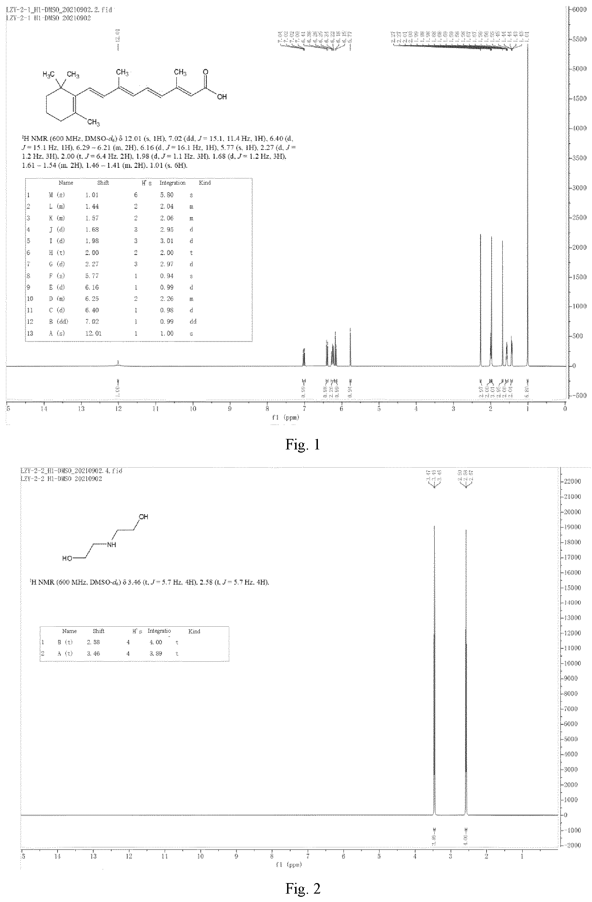

HNMR spectroscopy was performed on the retinoic acid, the diethanolamine, and the prepared retinoic acid-diethanolamine complex (2[DEA][RA]), as shown in Figs. 1 to 3 and the table below:

**Table A-2: Chemical shift of characteristic hydrogen atoms**

| **Molecule** | **Hydrogen position** | **Original chemical shift** | **Chemical shift in complex** |
|---|---|---|---|
| Retinoic acid | Hydroxy hydrogen | 12.01 | Disappear |
| | Carbon atom No. 2 | 7.02 | 6.78 |
| Diethanolamine | 1, 1' carbon atom | 2.58 | 2.76 |
| | 2, 2' carbon atom | 3.46 | 3.56 |

The results showed that the reactive hydrogen of the retinoic acid disappeared in the HNMR of 2[DEA][RA], indicating interaction between the retinoic acid and the diethanolamine. The carboxyl ortho-hydrogen atom of the retinoic acid underwent a chemical shift to a high field, with 7.02 → 6.78, indicating that the retinoic acid was anionized as a proton donor in the complex system, with the carboxyl oxygen atom as a charge center. Two pairs of methylene hydrogen of the diethanolamine underwent a chemical shift to a low field, with 2.58 → 2.76 and 3.46→3.56, respectively, indicating that the diethanolamine acted as a proton acceptor in the complex system. That is, the lone-pair electrons on the N atom were bound to free protons and cationized, with the N atom as the charge center.

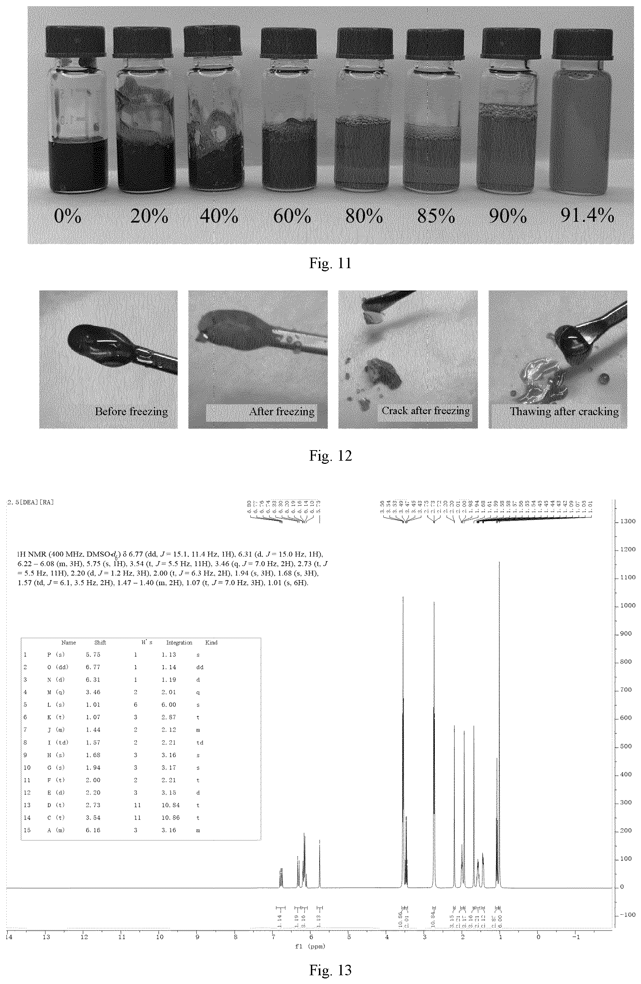

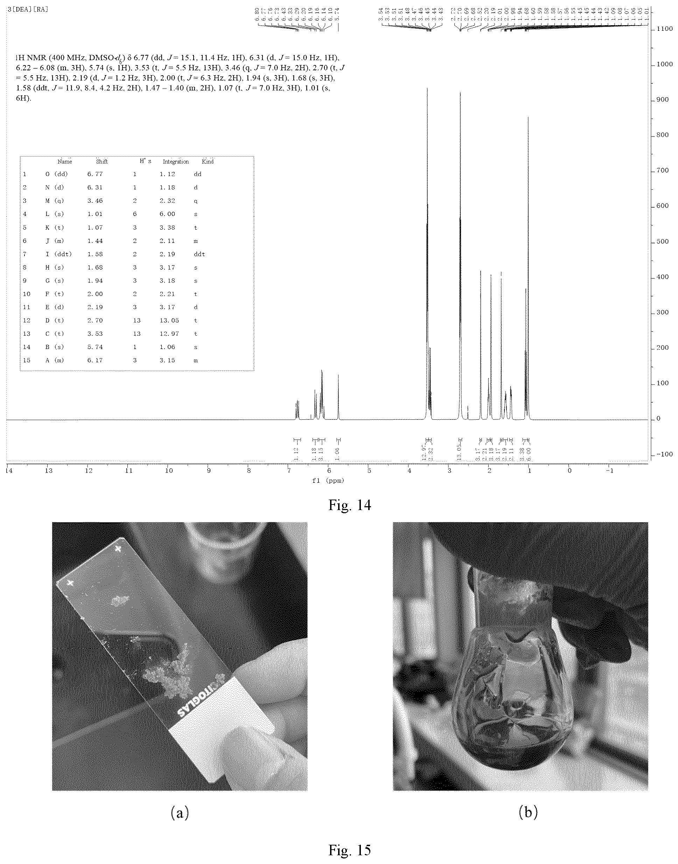

Figs. 13 and 14 show the HNMR spectra of complexes 2.5[DEA][RA] and 3[DEA][RA], respectively, with the same characteristic chemical shift changes as 2[DEA][RA]. It can be seen that the same intermolecular interaction occurs between the retinoic acid and the diethanolamine at a higher proportion, and stable liquid complexes can also be formed at room temperature.

### 1.4.2 Infrared (IR) spectrum

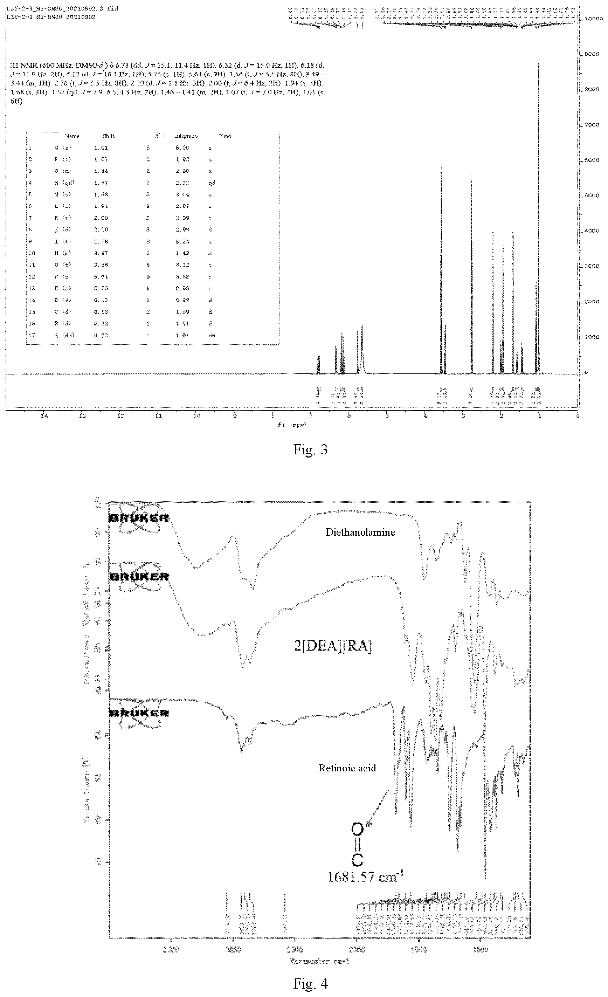

IR spectrometry was performed on the retinoic acid, the diethanolamine and the prepared 2[DEA][RA] (as shown in Fig. 4). The results showed that compared with the diethanolamine, the diethanolamine in 2[DEA][RA] had a less significant peak shift; and compared with the retinoic acid, the retinoic acid in 2[DEA][RA] showed that the characteristic frequency of C=O double bond (1681.57 cm-1) shifted to a lower wavenumber. These results indicated the intermolecular interaction between the retinoic acid and the diethanolamine.

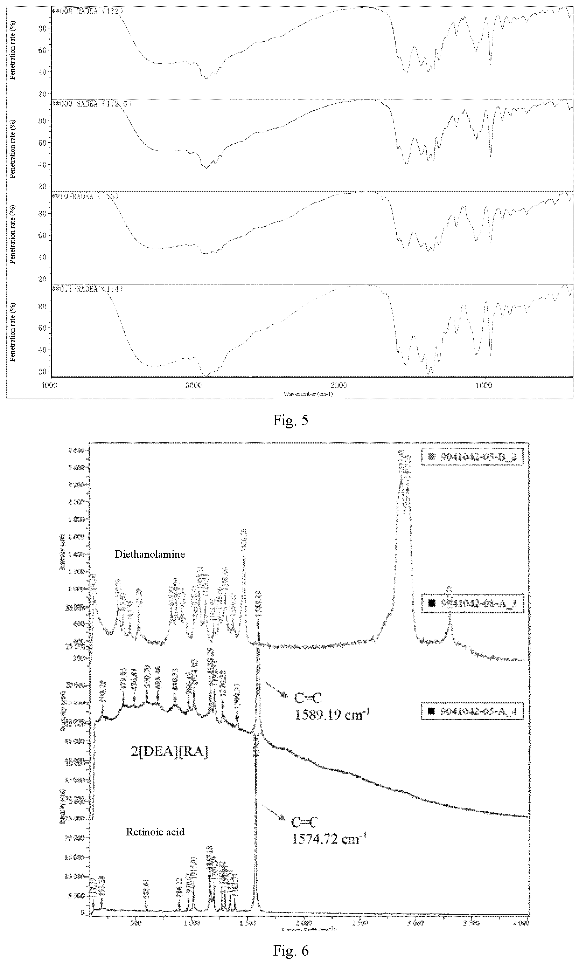

The complexes at different ratios were prepared at the molar ratios of RA:DEA = 1:2, 1:2.5, 1:3, and 1:4; and the infrared spectra of the complexes at different ratios were consistent (as shown in Fig. 5), and showed that the characteristic frequency of C=O double bond (1682 cm-1) shifted to a lower wavenumber, indicating the intermolecular interaction between the retinoic acid and the diethanolamine.

### 1.4.3 Raman spectrum

Raman spectrometry was performed on the retinoic acid, the diethanolamine and the prepared 2[DEA][RA] (as shown in Fig. 6). The results showed that the Raman spectrum of 2[DEA][RA] were significantly different from that of the diethanolamine, with the difference identifiable in contrast with the retinoic acid, and the characteristic frequency of C=C double bond (1574.72 cm⁻¹) shifted to 1589.19 cm⁻¹ at a high wavenumber, indicating the interaction between the retinoic acid and the diethanolamine.

### Preparation of retinoic acid-triethanolamine complex

Referring to the preparation process of 1.2 in Example 1, diethanolamine was replaced with triethanolamine, and the complex was prepared from retinoic acid and triethanolamine at a different ratio (as shown in the table below).

The results showed that a complex was formed when the molar ratio of the retinoic acid to the triethanolamine was 1:8, 1:9, or 1:10, and was hardly formed when the molar ratio was 1:(1~7) (see Fig. 15).

**Table B-1: Retinoic acid-triethanolamine complexes with different ligand ratios**

| **Name** | **Molar ratio (TEA: RA)** | **State at room temperature** |
|---|---|---|
| 7[TEA][RA] | 7:1 | Turbid liquid, and suspended yellow solid (RA precipitation) |
| 8[TEA][RA] | 8:1 | Transparent reddish-brown liquid |
| 9[TEA][RA] | 9:1 | Transparent reddish-brown liquid |
| 10[TEA][RA] | 10:1 | Transparent reddish-brown liquid |

### Study on General Physiochemical Properties of Retinoic Acid-Alcohol Amine Complex

The physiochemical properties of 2[DEA][RA] prepared according to 1.2.1 were studied as follows.

### 1.5 Moisture and residual solvent

Three batches of 2[DEA][RA] were tested using a Kar1 Fischer moisture tester, and the results showed that the moisture and residual solvent in the prepared complex were easy to remove.

**Table C-1: Water content of retinoic acid complex**

| **Complex batch** | **Batch 1** | **Batch 2** | **Batch 3** |
|---|---|---|---|
| **Moisture** | 0.38% | 0.44% | 0.41% |

### 1.6 Dynamic vapor sorption (DVS)

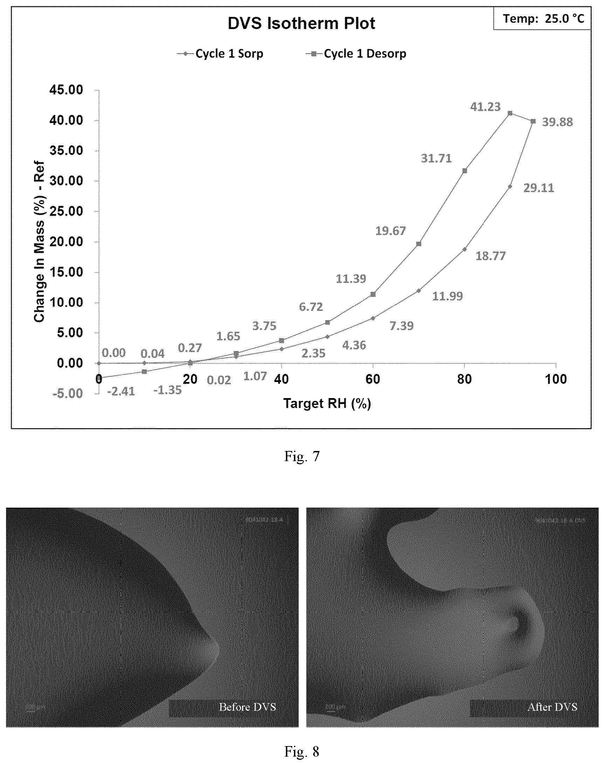

The DVS results showed (see Fig. 7) that 2[DEA][RA] had certain hygroscopicity. The PLM results (see Fig. 8) showed that 2[DEA][RA] before and after DVS did not contain crystalline particles, indicating that the complex had a low risk of retinoic acid precipitation in ambient humidity and was able to stably maintain liquid form.

### 1.7 Differential Scanning Calorimetry (DSC)

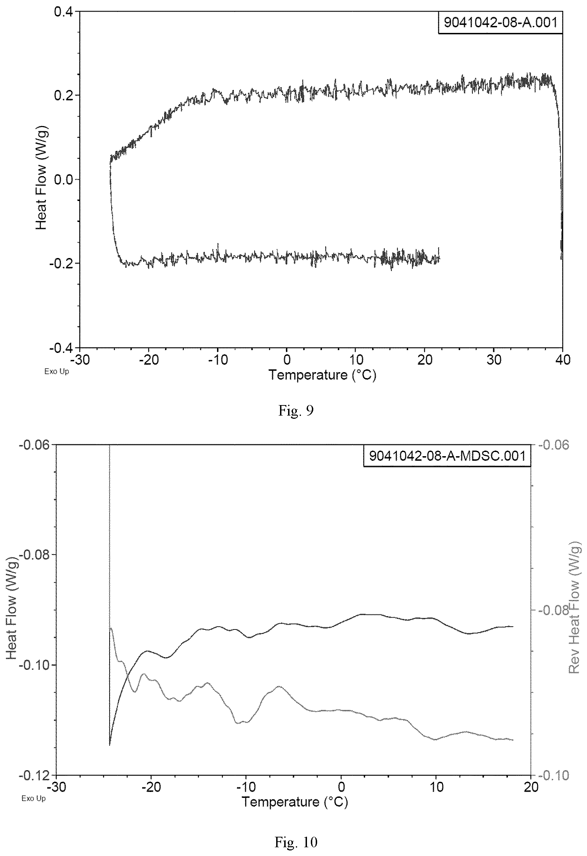

The DSC results (see Fig. 9) showed that 2[DEA][RA] had no exothermic or endothermic signal in the range of - 25°C ~ 40°C, indicating that the complex had no freezing point in this temperature range. The mDSC results (see Fig. 10) showed that the glass transition temperature of the complex was not observed in the range of -25°C ~ 25°C. This indicated that the complex had no risk of solidification and precipitation in this temperature range, and was able to maintain a stable liquid form.

### Water Solubility/Water Dilution Resistance of Retinoic Acid Complex

### 1.8 Test objective

Under the item of retinoic acid in volume II of Chinese Pharmacopoeia 2020, it is shown that the retinoic acid is extremely difficult to dissolve in water. Based on literature, it is known that the solubility of retinoic acid in water is only 1.06*10⁻⁶ mol/L (Ascenso A, Guedes R, Bernardino R, et al. Complexation and full characterization of the tretinoin and dimethyl-β-cyclodextrin complex. AAPS PharmSciTech. 2011;12(2):553-563. doi:10.1208/s12249-011-9612-3). The insoluble nature of retinoic acid poses a great challenge to the preparation process. However, a retinoic acid complex shows certain water solubility, which is conducive to the preparation of the drug product.

### 1.9 Test procedures

2[DEA][RA] was accurately weighed and was mixed by vortexing with different proportions of water, and the properties of the complexes with different water contents were observed; and after the water content exceeded 90%, water was continued to be added in small quantities at high frequency using a microsyringe for vortexing and mixing until each mixture became turbid, and each complex was weighed and calculated for the ultimate water content.

### 1.10 Results & Discussion

As shown in Fig. 11, when the water content was in the proportion range of 0~91.4% (w/w), 2[DEA][RA] was miscible with water (the liquid in the flask with a water content of ≤ 90% was clear and transparent). When the water content was too high (> 91.4%), the hydrogen bond in the water broken the complex system, leading to the precipitation of solid retinoic acid (the liquid in the flask with the water content of 91.4% was turbid). The complexes at other molar ratios were tested in water dilution resistance. The test results shown in the table below indicated that with the increase of DEA ratio, the water dilution resistance of the retinoic acid-diethanolamine complex increased.

**Table D-1: Water dilution resistance of retinoic acid-diethanolamine complexes at different ratios**

| **[RA]: [DEA]** | **1:2** | **1:2.5** | **1:3** | **1:4** |
|---|---|---|---|---|
| **Maximum water content (%)** | 91.4% | 92.5% | 93.7% | 94.1% |

### Induced Crystallization Test of Retinoic Acid complex

### 1.11 Test objective

In the practice of chemical synthesis process, it is often to form an amorphous crystalline or supercooled liquid during the recrystallization of a compound, with glassy appearance and clear oily form. This is similar to the characteristics of the complex of the present invention. However, this state is not stable, the compound will transform to its stable crystalline state when external conditions change or under the influence of a crystal seed. Therefore, in order to exclude this possibility and to further show that the complex prepared by the present invention has a stable liquid form, a series of induced challenge tests is proposed to carry out to obtain the solid form of the complex by a variety of means such as volatility test, stirring test, cooling-down test or the like to promote crystallization; and if the solid form cannot be obtained, it can be considered that the liquid form of the obtained complex is its stable form.

### 1.12 Volatility test

To investigate if a solid substance will be produced after the complete volatilization of the solvent at different temperatures under different solvent systems, in the presence or absence of a polymer. Here, the polymer is added for the purpose of adding a "crystal nucleus" to the system, so as to compare the effects of the presence and absence of the crystal nucleus; and different temperatures will affect the volatilization speed of the solvent and the crystallization behavior of a substance.

About 80 mg of 2[DEA][RA] was dissolved in the corresponding solvent to obtain a clear solution, which was then divided into 4 portions; a polymer was used as the "crystal nucleus", under the condition of no polymer, or polymer B added, or polymer C added, the solutions were allowed to volatilize at 5°C and 50°C for about 4 days; and the sample states were observed, and no substance in a solid form was observed except for the added polymer. The results are shown in the table below.

**Table E-1: Volatility test results**

| No. | Solvent system | Mass | Volume | Temper ature | Polymer | Results |
|---|---|---|---|---|---|---|
| Volatility test - A1 | acetone | 83.5 mg | 0.6 mL | 5 °C | No addition | Viscous yellowish-brown product |
| Volatility test -A2 | | | | | Polymer B | Viscous yellowish-brown product |
| Volatility test - A3 | | | | 50 °C | No addition | Viscous reddish-brown product |
| Volatility test - A4 | | | | | Polymer C | Viscous reddish-brown product |
| Volatility test - B1 | 2-MeTHF | 79.9 mg | 0.4 mL | 5 °C | No addition | Viscous yellowish-brown product |
| Volatility test - B2 | | | | | Polymer B | brown product |
| Volatility test - B3 | | | | 50 °C | No addition | Viscous reddish-brown product |
| Volatility test - B4 | | | | | Polymer C | Viscous reddish-brown product |
| Volatility test - C1 | MeOH/THF (5:1, v/v) | 80.0 mg | 0.4 mL | 5 °C | No addition | Viscous yellowish-brown product |
| Volatility test - C2 | | | | | Polymer B | Viscous yellowish-brown product |
| Volatility test - C3 | | | | 50 °C | No addition | Viscous reddish-brown product |
| Volatility test - C4 | | | | | Polymer C | Viscous reddish-brown product |
| Volatility test - D1 | EtOAc/ACN (10:1,v/v) | 79.6 mg | 0.4 mL | 5 °C | No addition | Viscous yellowish-brown product |
| Volatility test - D2 | | | | | Polymer B | Viscous yellowish-brown product |
| Volatility test - D3 | | | | 50 °C | No addition | Viscous reddish-brown product |
| Volatility test - D4 | | | | | Polymer C | Viscous reddish-brown product |

| | | | | | | |
|---|---|---|---|---|---|---|
| Polymer B: a mixture composed of five polymers, including polycaprolactone (PCL), polyethylene glycol (PEG), polymethyl methacrylate (PMMA), stearyl acrylate (SA), and hydroxyethyl cellulose (HEC), at an equal mass ratio. Polymer C: a mixture composed of four polymers, including polystyrene, polytetrafluoroethylene, polytribromostyrene, and polyvinyl stearate, at an equal mass ratio. | | | | | | |

### 1.13 Stirring test

0.3 mL of the corresponding solvent was added to about 20 mg of 2 [DEA] [RA] at room temperature (~22 °C) and stirred at the corresponding temperature for 3 days, to obtain a clear solution or observe oil formation, without any solid form obtained. The results are shown in the table below:

**Table E-2: Stirring test results**

| No. | Solvent system | Mass | Temperat ure | Results |
|---|---|---|---|---|
| Stirring test - A1 | EtOH | 20.8 mg | 5 °C | Clear at the beginning and still clear after stirring for 3 days |
| Stirring test - A2 | | 21.9 mg | -20 °C | Clear at the beginning and still clear after stirring for 3 days |
| Stirring test - B1 | DCM | 21.8 mg | 5 °C | Clear at the beginning and still clear after stirring for 3 days |
| Stirring test - B2 | | 21.5 mg | -20 °C | Clear at the beginning and still clear after stirring for 3 days |
| Stirring test - C1 | IPAc | 21.4 mg | 5 °C | Clear at the beginning and oily after stirring for 3 days |
| Stirring test - C2 | | 19.9 mg | -20 °C | Clear at the beginning and oily after stirring for 3 days |
| Stirring test - D1 | toluene | 21.9 mg | 5 °C | Clear at the beginning and still clear after stirring for 3 days |
| Stirring test - D2 | | 20.0 mg | -20 °C | Clear at the beginning and still clear after stirring for 3 days |
| Stirring test - E1 | MEK/ACN (3:2, v/v) | 19.9 mg | 5 °C | Clear at the beginning and still clear after stirring for 3 days |
| Stirring test - E2 | | 20.7 mg | -20 °C | Clear at the beginning and still clear after stirring for 3 days |
| Stirring test - F1 | THF/n-heptane (1:2, v/v) | 20.4 mg | 5 °C | Oily at the beginning and still oily after stirring for 3 days |
| Stirring test - F2 | | 20.7 mg | 50 °C | Oily at the beginning and still oily after stirring for 3 days |
| Stirring test - G1 | acetone/H2O (2:1, v/v) | 20.7 mg | 5 °C | Clear at the beginning and still clear after stirring for 3 days |
| Stirring test - G2 | | 21.1 mg | -20 °C | Clear at the beginning and still clear after stirring for 3 days |

### 1.14 Cooling-down test

About 40 mg of 2[DEA][RA] was dissolved in the corresponding solvent at 50°C, to obtain a clear solution, which was then divided into 2 portions, cooled down according to the corresponding procedure, and equilibrated for 3 days at low temperature, after which no solid form was obtained. The results are shown in the table below:

**Table E-3: Cooling-down test results**

| No. | Solvent system | Mass | Volume | Temperature | Results |
|---|---|---|---|---|---|
| Cooling-down test - A1 | n-propanol | 40.3 mg | 0.2ml | 50°C→5°C | Clear |
| Cooling-down test - A2 | | | | 50°C→-20°C | Clear |
| Cooling-down test - C1 | 1,4-dioxane | 41.9 mg | 0.2mL | 50°C→5°C | Clear |
| Cooling-down test - C2 | | | | 50°C→-20°C | Frozen, yellow, untransparent * |
| Cooling-down test - D1 | DMSO | 42.2 mg | 0.2mL | 50°C→5°C | Clear |
| Cooling-down test - D2 | | | | 50°C→-20°C | Frozen, yellow, semitransparent * |
| Cooling-down test - E1 | acetone/ACN (1:1, v/v) | 41.1 mg | 0.2mL | 50°C→5°C | Oily yellowish-brown product |
| Cooling-down test - E2 | | | | 50°C→-20°C | Few oily yellowish-brown product |
| Cooling-down test -B | Acetic acid | 40.4 mg of the complex was dissolved in 1.4 mL of solvent without clarification, and thus stirred for approximately 7 days at 50°C, to obtain a solid crystal form that was consistent with the starting retinoic acid. | | | |

| | | | | | |
|---|---|---|---|---|---|
| 50°C → 5°C: The resulting clear solution was equilibrated for 30 min at 50°C, cooled down over 450 min to 5°C, and equilibrated for 3 days at 5°C. 50°C → -20°C: The clear solution obtained at 50 °C was quickly transferred to a place at -20 °C and equilibrated for 3 days at -20°C. *: Thaw immediately after removal. | | | | | |

### 1.15 Liquid Nitrogen Freezing Test

About 50 mg of 2[DEA][RA] was obtained with a measuring scoop, then placed, together with the scoop, into liquid nitrogen (2[DEA][RA] was a transparent reddish-brown liquid before freezing), frozen for about 1 min and then taken out to obtain a transparent reddish-brown solid (hard and brittle), which, however, thawed immediately after rewarming (room temperature: ~22 °C) (return to the transparent reddish-brown liquid), and no solid form was found. The test procedures were shown in Fig. 12.

### 1.16 Anti-solvent addition test

The corresponding volume of positive solvent was added to an appropriate amount of 2[DEA][RA] to obtain a clear solution. If there were 2 types of corresponding anti-solvents, the clear solution was divided into 2 portions. At room temperature (~22°C), the anti-solvent was slowly added to the clear solution over stirring until a solid was precipitated (the maximum volume of anti-solvent added was 1.5 mL), and if the sold was precipitated, adding the anti-solvent was stopped; and the anti-solvent was stirred for about 4 days at room temperature to obtain a clear solution or observe oil formation, without any solid form obtained. The results are shown in the table below.

**Table E-4: Anti-solvent addition test**

| No. | Positive solvent | Mass | Volume (positive solvent) | Anti-solvent | Volume (anti-solvent) | Results |
|---|---|---|---|---|---|---|
| Anti-solvent addition test - A | DMSO | 20.1 mg | 0.05 mL | ACN | 1.5 mL | Clear |
| Anti-solvent addition test - B1 | n-propanol | 40.4 mg | 0.3 mL | ACN | 1.5 mL | Clear |
| Anti-solvent addition test - B2 | | | | n-heptane | 1.5 mL | Clear |
| Anti-solvent addition test - C1 | acetone | 39.4 mg | 0.1 mL | ACN | *0.15* mL | Oily |
| Anti-solvent addition test - C2 | | | | n-heptane | *0.15* mL | Oily |
| Anti-solvent addition test - D1 | THF | 41.8 mg | 0.1 mL | ACN | 1.5 mL | Clear |
| Anti-solvent addition test - D2 | | | | n-heptane | 0.05 mL | Oily |
| Anti-solvent addition test - E1 | IPA | 39.6 mg | 0.2mL | ACN | 1.5 mL | Clear |
| Anti-solvent addition test - E2 | | | | n-heptane | 0.85 mL | Oily |

### 1.17 Summary of induced crystallization test

A total of 49 induced crystallization tests (including volatilization, stirring, cooling down and anti-solvent addition, and liquid nitrogen freezing) were performed by starting with the retinoic acid-diethanolamine complex. The reaction solvent covered different classes of single solvents or bi-solvent combinations, and the reaction temperature ranged from -20°C to 50°C (the temperature of the liquid nitrogen freezing test was -196°C). No solid form was obtained in all the tests. The results showed that it was difficult for the retinoic acid-diethanolamine complex to exist in solid form, that is, the liquid form of the complex was its stable form at room temperature.

### Investigation of Penetration Promotion Effect of Retinoic Acid Complex

### 1.18 Comparison of penetration promotion effects of retinoic acid-diethanolamine complexes at different concentrations

The skin penetration effect of the retinoic acid was studied by in vitro transdermal tests in which 2[DEA][RA] was mixed with different amounts of water to prepare complex solutions of 10%, 20% and 40% (w/w), respectively. The results showed that the skin penetration effect of the retinoic acid increased with the increase of water content.

**Table F-1: Comparison of skin penetration effects of 2[DEA][RA]s at different concentrations**

| **Sample (complex concentration (w/w)%)** | **Unit area penetration amount, µg/cm²** | | | | |
|---|---|---|---|---|---|
| | **1** | **2** | **3** | **Mean** | **RSD%** |
| 2[DEA][RA]-10% | 27.97 | 8.85 | 13.71 | 16.84 | 59.01 |
| 2[DEA][RA]-20% | 11.30 | 5.38 | 18.31 | 11.66 | 55.53 |
| 2[DEA][RA]-40% | 8.74 | 7.51 | 6.31 | 7.52 | 16.16 |

### 1.19 Transdermal test for the complex

### 1.19.1 Preparation of complex solutions

A certain amount of the complex 2[DEA][RA] was weighed and dissolved in purified water (so that the concentration of the complex in the aqueous solution was 10% (w/w)), stirred to a uniform state, and vortexed for 3 min to mix the sample evenly. Based on the retinoic acid, the concentration of the prepared aqueous solution of the complex was 5.88% (w/w).

### 1.19.2 Preparation of retinoic acid paste

20 g of o/w matrix was prepared according to the prescription below for later use.

| **Ingredient** | | **Manufacturer** | **Ratio** |
|---|---|---|---|
| **Water phase** | Water | Self-made | 48.12% |
| | Propanediol | Sinopharm | 5% |
| | Tween 80 | Sigma | 5% |
| **Oil phase** | Retinoic acid | Aladdin | 5.88% |
| | Medium-chain triglyceride | Gattefosse | 20% |
| | Mono- and di-glyceryl stearate | Gattefosse | 3% |
| | Hexadecanol | Sinopharm | 6% |
| | Octadecanol | Sinopharm | 6% |

### Process:

An aqueous-phase material was weighed according to the prescription, and mixed evenly for later use; an oil-phase material was weighed according to the prescription, heated to 80°C to reach a molten state and mixed evenly; the aqueous-phase material was added while the oil-phase material was hot, and then homogenized for 2 min at 20000 rpm with a high-speed shear dispersion emulsifier to form a light-yellow emulsion, which was let cool down to room temperature.

### 1.19.3 Basic information of transdermal test

| | |
|---|---|
| **1. Device information** | TK-24BL |
| **2. Material** | Dorsal skin of Bama minipigs (TEWL < 20 g/m²h, preferably skinwith fewer pores)* |
| **3. Receiving solution** | PBS of pH 7.4 (with 1% (w/v) bovine serum albumin) |
| **4. Test conditions** | 32°C, stirring speed: 400 rpm, with protection from light during the test |
| **5. Sampling time point** | 6h |
| **6. Dosage^{**}** | 0.5 ml***, n=3 (parallel tests in triplicate) |

| | |
|---|---|
| *: Before administration, the skin should be placed on a diffusion tank for equilibration for 1 h, and the TEWL value should be measured; **: during administration, it should be ensured that the paste was applied evenly to come into full contact with the skin, so as to avoid air bubbles; and ***: the actual dosage was measured by the balance. | |

### 1.19.4 Test procedures:

**(1) Preparation of test:** samples and receiving solutions were prepared in advance; and the pig skin, preferably the skin with fewer pores, was prepared into an appropriate size, and then weighed, with the weight difference less than 0.05 g.
**(2) Skin equilibration:** a Franz diffusion pool was assembled, and the skin was carefully fixed; the receiving solution was added, and the air bubbles were removed to ensure that the receiving solution was in full contact with the lower surface of the skin; after the temperature of the diffuser reached the preset temperature, the skin was equilibrated for 1 h; and the TEWL value was measured, which should be less than 20 g/m²h.
**(3) Test:** about 0.5 ml was administered, and the paste should be applied evenly to come into full contact with the skin; and the actual dosage was obtained by weighing with the balance.
**(4) Sampling:** after 6 hours, the test was completed, and the receiving solution and skin were collected and treated.

### 1.19.5 Sample handling

### (1) Receiving solution:

After the transdermal test, 1.5 ml of the receiving solution was pipetted and centrifuged for 15 min at 12000 rpm, and the supernatant was detected by HPLC.

### (2) Skin extract:

After the transdermal test was completed, the skin was wiped with dust-free paper to remove the sample on the surface, and rinsed with no less than 5 ml of PBS; the skin was carefully removed and placed on a piece of filter paper to absorb water on both sides; an adhesive tape was applied to the upper surface of the skin 6 times to remove the stratum corneum; and then, the upper surface of the skin was carefully rubbed with a cotton ball wetted with ethanol.

After the pressed periphery of the skin was cut off, the skin was cut into pieces, and placed in a 5 ml centrifuge tube; 1 ml of methanol was added to allow for full immersion of the skin pieces; ultrasonic extraction was performed for 60 min; after the ultrasonic extraction, 0.8 ml of the extract was pipetted, centrifuged for 15 min at 12000 rpm, and the supernatant was detected by HPLC.

### 1.19.6 Test Results

### (1) HPLC results of skin penetration test

No retinoic acid was detected in the receiving solution. The amount of retinoic acid retained in the skin was measured, with the results as follows:
**Retention volume of retinoic acid in skin (unit area penetration amount: µg/cm²)**

| **Sample** | **1** | **2** | **3** | **Average Value** | **RSD%** |
|---|---|---|---|---|---|
| 2[DEA][RA] water solution | 27.97 | 8.85 | 13.71 | 16.84 | 59.01 |
| RA ointment | 1.13 | 0.90 | 1.25 | 1.09 | 16.25 |

As shown in the table above, the retention volume in skin for the 2[DEA][RA] aqueous solution group was significantly higher than that in the RA paste group, indicating that 2[DEA][RA] had a significant penetration promotion effect.

### (2) Skin staining after transdermal test (removing stratum corneum and wiping with ethanol)

At the end of the transdermal test, the surface of the skin was washed, the adhesive tape was applied to remove the stratum corneum, and the skin was rubbed with ethanol to remove the sample remaining on the surface of the skin as much as possible. The test results showed that compared with the RA paste group, the skin of the 2[DEA][RA] aqueous solution group was significantly stained and showed the characteristic yellow color of the retinoic acid. It indicated that 2[DEA][RA] significantly improved the skin penetration effect of the retinoic acid. It should be noted that the actual concentrations of the used retinoic acid drug product (0.025%, 0.05%, 0.1%) were far below the concentration of the test sample (5.88%), and thus, there was no need to worry about the side effect of skin staining in actual use. In this test, the purpose of selecting the high-concentration sample was to meet the limit of quantification required by HPLC and also to more easily verify the penetration promotion effect of the 2[DEA][RA].

The embodiments of the present invention are illustrated above. However, the present invention is not limited to the embodiments above. Within the essence and principle of the present invention, any modifications, equivalent substitutions, improvements and the like should be construed as falling within the protection scope of the present invention.

## Claims

1. A retinoic acid-alcohol amine complex, **characterized in that** said complex comprises a retinoic acid and an alcohol amine compound.

2. The complex according to claim 1, **characterized in that** said alcohol amine compound is selected from formula I as follows: 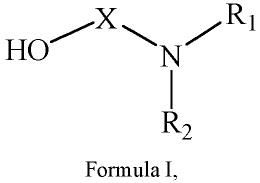 wherein X is selected from C₁₋₆ alkyl, preferably, from -CH₂-CH₂-, -CH₂-CH₂-CH₂- and 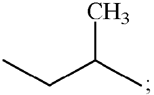 R₁ **and** R₂ are each independently selected from H, C₁₋₆ alkyl and C₁₋₆ alkyl-OH; and preferably, said formula I is selected from diethanolamine, triethanolamine, monoethanolamine(2-hydroxyethylamine), N-methyldiethanolamine, n-propanolamine, isopropanolamine, diisopropanolamine, triisopropanolamine, n-butanolamine, dibutolamine, and isobutanolamine.

3. The complex according to claim 1, **characterized in that** molar ratio of said alcohol amine compound to the retinoic acid is (1-100):1, for example, 2:1, 2.5:1, 3:1, 3.5:1, 4:1, 4.5:1, 5:1, 5.5:1, 6:1,, 6.5:1, 7:1, or (1-10):1, more preferably, (1-5):1.

4. The complex according to claim 1, **characterized in that** said complex is in a liquid form at room temperature.

5. The complex according to claim 1, **characterized in that** said complex, said retinoic acid can exist partially or wholly in anionic form, and said alcohol amine compound can exist partially or wholly in cationic form.

6. The complex according to claim 5, **characterized in that** said retinoic acid exists in [A⁻] form as follows:

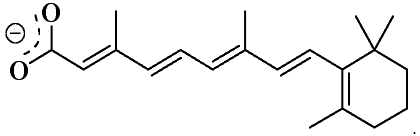

7. The complex according to claim 5, **characterized in that** said alcohol amine compound exists in [B⁺] form as shown in formula II as follows: 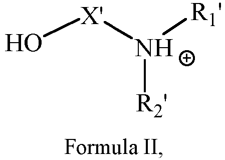
wherein X' is selected from C1-6 alkyl, preferably, from -CH₂-CH₂-, -CH₂-CH₂-CH₂- and 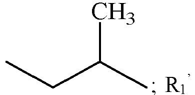 and R₂^{'} are each independently selected from H, C₁₋₆ alkyl and C₁₋₆ alkyl-OH; and
preferably, said formula II is selected from diethanolamine cation, triethanolamine cation, monoethanolamine cation, N-methyldiethanolamine cation, n-propanolamine cation, isopropanolamine cation, diisopropanolamine cation, triisopropanolamine cation, n-butanolamine cation, dibutolamine cation, and isobutanolamine cation.

8. A preparation method for said complex according to any one of claims 1-7, comprising the step of:
reacting the retinoic acid with the alcohol amine compound to obtain said complex, preferably, molar ratio of said alcohol amine compound to the retinoic acid is (1-100):1.

9. A composition, **characterized in that** said composition comprises the retinoic acid-alcohol amine complex according to any one of claims 1-7.

10. An application of the retinoic acid-alcohol amine complex according to any one of claims 1-7 or the composition according to claim 9 in preparation of a pharmaceutical preparation for treatment of skin diseases and non-skin tumors, preferably, said pharmaceutical preparation is used for treatment of skin diseases.
